# EUROPEAN PATENT APPLICATION

(11) **EP 4 390 961 A1**
(43) Date of publication of application: **26.06.2024**
(21) Application number: 23217554.7
(22) Date of filing: 18.12.2023
(51) Int. Cl.: G16H 40/20, G06Q 10/06

(54) **HEALTHCARE FACILITY SCHEDULING**

(30) Priority: 22.12.2022 US 202218145089
(71) Applicant: TeleTracking Technologies, Inc., Pittsburgh, PA 15222 (US)
(72) Inventor: DEGRANDPRE, John, Pittsburgh, Pennsylvania 15222 (US); MABEE, Alison, Pittsburgh, Pennsylvania 15222 (US); JAKOPIN, Daniel, Pittsburgh, Pennsylvania 15222 (US); BHURANI, Deepak, Pittsburgh, Pennsylvania 15222 (US); BAPURI, Rajat, Pittsburgh, Pennsylvania 15222 (US); KUMAR, Chethan, Pittsburgh, Pennsylvania 15222 (US); SINGH, Sumer, Pittsburgh, Pennsylvania 15222 (US)
(74) Representative: Sandri, Sandro

(57) **Abstract**

One embodiment provides a method, the method including: obtaining patient information for at least one patient, wherein the patient information identifies an identifier of the at least one patient; identifying, based upon the patient information, at least one procedure to be performed for the patient at the healthcare facility; identifying a plurality of events corresponding to the at least one procedure and locations within the healthcare facility for at least a subset of the plurality of events; identifying at least one healthcare professional associated with at least one of the plurality of events; and automatically generating a schedule for the healthcare facility based upon the plurality of events and the at least one healthcare professional. Other aspects are described and claimed.

## Description

### BACKGROUND

Healthcare systems are a critical part of a community's infrastructure. Patients enter healthcare systems everyday for various things, for example, emergencies, scheduled procedures, testing, consultations, and/or the like. Some of these visits may consist of different steps or events, such as registration, pre-procedure preparation, procedure performance, post-procedure recovery, different testing locations, procedure consultation, and/or the like. As such each of the steps or events needs to be scheduled so that the patient can flow through the procedure process without hiccup. Additionally, any objects (e.g., healthcare equipment, procedure objects, testing equipment, etc.), healthcare professionals (e.g., surgeons, doctors, nurses, physician assistants, etc.), other people (e.g., transport staff, janitorial staff, etc.), need to be scheduled so that the procedure process will flow smoothly and each of the locations needed for the procedure will be prepared for the patient and can quickly be turned around for the next patient that may need the space.

### BRIEF SUMMARY

In summary, one aspect provides a method, the method including the steps described at claim 1. The dependent claims outline advantageous ways of carrying out the method.

Another aspect provides a system, the system including: a processor; a memory device that stores instructions that, when executed by the processor, causes the system to carry out a method comprising the steps described in any one of the method claims.

A further aspect provides a product, the product including: a computer-readable storage device that stores executable code that, when executed by a processor, causes the product to: obtain, at a scheduling system of a healthcare facility, patient information for at least one patient, wherein the patient information identifies an identifier of the at least one patient; identify, using the scheduling system and based upon the patient information, at least one procedure to be performed for the patient at the healthcare facility; identify, using the scheduling system, a plurality of events corresponding to the at least one procedure and locations within the healthcare facility for at least a subset of the plurality of events; identify, using the scheduling system, at least one healthcare professional associated with at least one of the plurality of events; and automatically generate, using the scheduling system, a schedule for the healthcare facility based upon the plurality of events and the at least one healthcare professional.

The foregoing is a summary and thus may contain simplifications, generalizations, and omissions of detail; consequently, those skilled in the art will appreciate that the summary is illustrative only and is not intended to be in any way limiting.

For a better understanding of the embodiments, together with other and further features and advantages thereof, reference is made to the following description, taken in conjunction with the accompanying drawings. The scope of the invention will be pointed out in the appended claims.

### BRIEF DESCRIPTION OF THE SEVERAL VIEWS OF THE DRAWINGS

FIG. 1 illustrates an example method of simplified user management functionality for managing users within a software application.
FIG. 2 illustrates an example of device circuitry.

### DETAILED DESCRIPTION

Generally, scheduling the events, objects, healthcare professionals, and/or the like, of a procedure process is a manual process. Additionally, scheduling many of the events or locations needed by a procedure process are triggered by the completion of a previous event of the procedure process. This means that a patient may have to wait at a previous procedure process step or location until the next procedure process step or location is prepared for the patient. For example, once a surgeon is finished or almost finished with a surgery, the recovery room readiness step may be triggered. However, if there is not a recovery room that is ready for the patient, the patient may have to wait in the operating room until the recovery room has been prepared for the patient. Not only does this delay the patient's process, but it also causes the operating room to be unnecessarily occupied when it could be being tuned over for the next surgery. This means that any subsequent surgery that was scheduled gets delayed.

Additionally, the manually scheduling is based upon an average length of time for a particular procedure step or event. However, an average means that sometimes when the procedure step is performed, it takes longer than the average and sometimes it is shorter than the average. Using this average length means that sometimes the procedure step will take longer, thereby pushing into any subsequent procedure steps for the patient and also pushing into any subsequent procedures within the procedure location or performed by the healthcare professional. Additionally, in the event that the procedure step is performed in a shorter length of time, valuable time is wasted with a deadtime for the healthcare professional and/or procedure location that could have been used for performing another procedure.

Accordingly, the described system and method provides a system and method for automatically generating a schedule for a healthcare facility, which may include either a patient schedule, a healthcare professional schedule, a location schedule, a support personnel schedule, and/or the like, utilizing historical information regarding the patient, healthcare professional, location, support personnel, and/or the like, to optimize the scheduling. The scheduling system of a healthcare facility obtains patient information for at least one patient. The patient information includes an identifier that allows for identification of the patient. The patient information may be received when a patient registers or checks-in for a procedure, imported or accessed from another system associated with the healthcare facility, a combination thereof, and/or the like. From the patient information, the scheduling system can identify at least one procedure that is to be performed for the patient at the healthcare facility.

Based upon the identified at least one procedure, the scheduling system can identify a plurality of events or steps of the at least one procedure. In other words, the system identifies what tasks, events, or steps have to be performed to complete or perform the procedure. For example, the system may identify pre-procedure steps, procedure steps, and post-procedure steps. The granularity of the steps or events may be different for different healthcare professionals, healthcare facilities, procedures, patients, and/or the like.

Once the events or steps are identified, the scheduling system can identify at least one healthcare professional that is associated with at least one of the events. The healthcare professional may include one or more doctors, nurses, physician's assistants, clinicians, and/or the like, that may assist in performing a step or event of the procedure. For example, the healthcare professional may be a particular surgeon who will be performing a surgery for the patient. Additionally, the system may identify any support personnel, healthcare system locations, healthcare objects, and/or the like, that may be associated with a particular event or step within the procedure. It should be noted that none or more than one healthcare professional, support personnel, healthcare system location, healthcare object, and/or the like, can be associated with any individual event or step.

From the identified events and the healthcare professional associated with events of the procedure, the scheduling system can automatically generate a schedule for the healthcare facility. The schedule may be generated for the patient, a healthcare professional, a support personnel, a location within the healthcare system, a healthcare object, and/or the like. With the inclusion of multiple patients, full day schedules can be generated for the healthcare facility and staff, locations, and healthcare objects within the location. The scheduling can be performed based upon historical information related to the patient, patients having a similarity to the patient, the healthcare professional or other personnel performing a step of the procedure, the healthcare object used to perform a step of the procedure, and/or the like. Using this historical information allows for a more accurate and optimized scheduling, thereby reducing the amount of dead time and/or waiting for a subsequent event, thereby making a more streamlined and pleasant experience for a patient within the healthcare system and less frustration for those healthcare professionals or other healthcare personnel that are responsible for performing steps of the procedure.

The described system and method will be discussed with respect to a healthcare system or healthcare facility for ease of readability. Additionally, for simplicity, the term "hospital" may be used here throughout. However, it should be understood by one skilled in the art that this term may refer to any healthcare system or facility, for example, long-term care facility, emergency department, healthcare staffing area, hospital facility including multiple departments within a healthcare campus or affiliations, affiliated or otherwise connected healthcare systems, and the like. However, the fact that this disclosure is discussed with respect to a healthcare system or hospital is not intended to limit this disclosure to only scheduling within a healthcare system. As can be understood by one skilled in the art, the disclosed systems and methods can be applied to any entity that coordinates and/or generates daily, weekly, or other periodic schedules that are based upon incoming people, objects, and/or the like.

Additionally, the term "procedure" will be used throughout this disclosure. However, this is intended to encompass any type of procedure that may be performed in a healthcare system including, but not limited to, emergencies, scheduled procedures, testing, consultations, and/or the like. Additionally, to increase readability, the disclosure will discuss a patient entering the healthcare system for a single procedure. However, it should be understood that the described system can be utilized when a patient enters a healthcare system for more than one procedure, overlapping procedures (e.g., the same event or step can be utilized in multiple procedures for the patient, etc.), and/or the like. Additionally, procedures may occur over multiple days for the patient. Events or steps within the procedure refer to different steps, processes, or events, within a procedure that are required for the procedure to be completed. The term "event" will be generally used here throughout to refer to these steps, processes, or events within the procedure. Events may include those that occur before the procedure, those that include during the procedure, and/or events that occur after the procedure.

The illustrated example embodiments will be best understood by reference to the figures. The following description is intended only by way of example, and simply illustrates certain example embodiments.

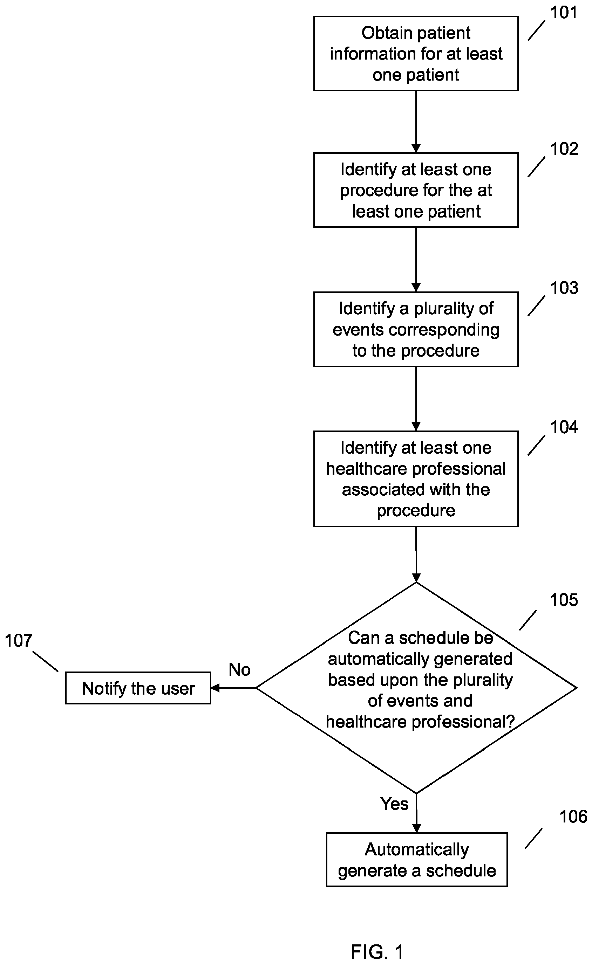

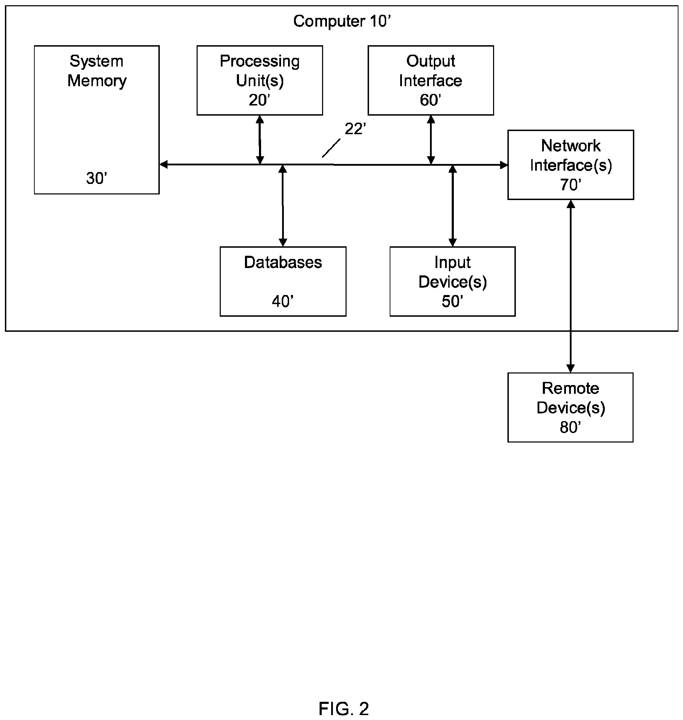

FIG. 1 illustrates a method for automatically generating a schedule for a healthcare system based upon a procedure to be performed for a patient and a healthcare professional that is associated with at least one event within the procedure. The method may be implemented on a system which includes a processor, memory device, output devices (e.g., display device, printer, etc.), input devices (e.g., keyboard, touch screen, mouse, microphones, sensors, biometric scanners, etc.), image capture devices, and/or other components, for example, those discussed in connection with FIG. 2. While the system may include known hardware and software components and/or hardware and software components developed in the future, the system itself is specifically programmed to perform the functions as described herein to provide a scheduling system for a healthcare facility that can automatically generate schedules for the healthcare system. Additionally, the scheduling system includes modules and features that are unique to the described system.

The healthcare system scheduling system, also referred to also as a scheduling system, may include or have access to network devices. The network devices may facilitate communication between the healthcare system scheduling system and another device, component, facility, healthcare system, and/or the like. For example, the scheduling system may utilize a network device to communicate with the healthcare systems applications, programs, and/or systems, that are providing and receiving transmissions.

The healthcare facility scheduling system is provided that allows for interface between one and more users and other systems within the healthcare facility. The scheduling system may communicate and transmit information with other hospital systems that may provide information regarding patients, healthcare professionals, hospital objects, support personnel or departments, and/or the like. For example, the scheduling system may interface with an object tracking system, patient registration system, employee system, support personnel or department system, and/or the like. Thus, the scheduling system can pull information from each of the interconnected or accessible systems to optimize schedules for the healthcare facility. The scheduling system can also transmit information to one or more of the interconnected or accessible systems.

The scheduling system may be a background application that does not have an associated display or monitor. Rather, the scheduling system, when accessed by a user will provide instructions to generate and display a graphical user interface on a display device utilized by the user. Thus, the scheduling system may not have its own display device, but does have the ability to generate and display a graphical user interface. The graphical user interface is updated and managed based upon instructions provided by the scheduling system. In other words, the scheduling system generates and transmits instructions to create and update the graphical user interface. Alternatively, or additionally, the scheduling system may be a standalone system that has an associated display device. In this case, the scheduling system still provides instructions for generating a graphical user interface as further described herein.

The scheduling system may also have a standalone system and may also be accessible through other computing devices. For example, the scheduling system may be a standalone system that can be accessed by a user and also may be an application that is accessible by a user on another computing device. The scheduling system may be accessible using any type of computing device, for example, personal computer, laptop computer, smartphone, tablet, smartwatch, head-mounted display, smart television or other smart appliance, and/or the like.

The graphical user interface may provide a user with the ability to access the schedule(s) generated by the scheduling system. Thus, the graphical user interface displays a generated scheduling, a progress of generating a scheduling, errors or alerts associated with generating a schedule, and/or the like. The scheduling and information associated with the schedule may be the default view when the graphical user interface is first accessed. Alternatively, or as configured by the system or user, the schedule and associated information may be displayed when a user interfaces with a scheduling icon. It should be noted that different users may configure the graphical user interface per their preferences. Thus, the graphical user interface layout and configuration may be different between users. How much a user can configure the layout may be restricted or set by a system administrator and/or the like. Additionally, different users or different user roles may have different levels of access, which may also change how and what information is displayed.

The graphical user interface may also provide different icons that allow a user to configure the system, including setting user or system preferences or defaults, identifying automatic notification recipients, identifying users having access to different portions of the scheduling system, providing interface information to interface with different hospital systems, facilitating updates of the system, and/or any number of other configuration options. The graphical user interface may also provide different icons that allow the user to provide input to the scheduling system, for example, modifying a generated schedule, viewing historical information, manipulating views provided to users, and/or any number of other input options.

The graphical user interface may include one or more graphical elements. The graphical elements may include user input fields that facilitate the generation of a schedule. The user input fields may also allow a user to provide input to view pending schedules, historical schedules, statistics of schedules (e.g., a frequency of scheduling of a particular resource (e.g., healthcare professional, support department or personnel, healthcare object, etc.), an accuracy of generated schedules, a frequency of scheduling overrun or overlap, a frequency of deadtime in schedules, etc.), and/or the like. Input fields may include radial selector buttons, pull-down menus, free-form fields, structured input fields, selection boxes, and/or any other type of input field. The graphical elements may include icons that a user can interact with.

Some icons may also be display icons that display information. The display icons may be static icons that have static visual elements. The display icons may be dynamic icons that have dynamic visual elements that change based upon an update in information displayed on the icon, periodically, based upon a condition being met, and/or the like. For example, the graphical element may be a display icon that displays the status of a schedule generation. As the status is updated, the graphical element is updated to reflect the new status. In other words, the graphical element is iteratively updated based upon changes to the status of the schedule generation. As should be understood, there may be other dynamic graphical elements, and this is only an example of one. For example, other dynamic graphical elements may include other status indicators, statistic indicators, and/or the like.

At 101 patient information for at least one patient may be obtained at the scheduling system. Obtaining the patient information may include accessing one or more of the interconnected or accessible systems. Obtaining the patient information may also include receiving the patient information from one or more of the interconnected or accessible systems. Obtaining the patient information may also be performed using a combination of techniques. For example, the scheduling system may receive patient information from one system and may use some of the received information to access a second or other subsequent system to obtain additional patient information.

One example of obtaining patient information includes the scheduling system importing patient information from a patient schedule or application. For example, when the patient schedules a procedure, the patient may have access to the scheduling information through an application. The scheduling system may be able to access the patient schedule from the application and may then retrieve additional patient information that is included or saved within the application, for example, a name, system patient identifier, and/or the like. Additionally, or alternatively, the patient applications are generally updated via a patient scheduling hub or application. The scheduling system could access that patient scheduling hub or application and pull scheduled patients and patient information from the hub or application or a system connected to the hub or application.

Another example of obtaining patient information includes the scheduling system obtaining patient information from patient registration information. The patient registration information may be provided by the patient when the patient registers for the procedure, for example, at the registration desk, registration kiosk, and/or the like. The patient registration information may also be pulled from a patient registration system that may contain information related to patients who have registered at the healthcare facility and/or patients who will register at the healthcare facility.

Another example of obtaining patient information includes importing patient information from other systems. For example, if a patient is transferred from one hospital department to another hospital department, the patient information may be obtained from the hospital system that includes the patient information. As another example, as a patient moves through the hospital or healthcare facility, patient tracking information may be captured. The scheduling system can then obtain this information to capture the patient information. The patient information may also be obtained by accessing a patient identification object or other object associated with or corresponding to the patient, for example, a patient identification bracelet, badge, or other identification object that is scanned and the information is pulled into the system, a bed that is assigned to the patient and is tracked throughout the healthcare facility, and/or the like.

The described techniques for obtaining the patient information are merely examples and are not intended to limit the scope of this disclosure to only the described techniques. The scheduling system can also obtain patient information using a combination of the described techniques or other techniques for obtaining information. For example, the system may obtain patient identification information from a registration system and then may use that information to access another system to obtain additional patient information.

The patient information may include at least one patient identifier of the patient that allows for the patient to be identified. The patient identifier may include a patient name, an alias, a patient identification number, and/or any other identifier that allows for the patient to be identified. The patient information may also include or identify other characteristics of the patient, for example, age, gender, geographic location, or other characteristics. The patient information may also include health characteristics of the patient, for example, allergies, accommodations needed, special instructions, health conditions, and/or the like. The patient information may also include information regarding historical visits of the patient, for example, prior procedures and results, complications, schedules, and/or the like associated with the prior procedures. Other patient information may be possible and contemplated and may be based upon needs of the hospital or scheduling system.

At 102, the scheduling system identifies at least one procedure to be performed for the patient at the healthcare facility. To identify the procedure, the system may utilize the patient information to identify what procedure the patient is scheduled for, what procedure the patient has checked-in for, what procedure the patient has indicated is needed, and/or the like. In the event that the procedure is not identified when the patient information is identified, the system may use the patient information to access a system to identify the procedure. For example, the system may access a procedure system and search the procedure system using one or more pieces of patient information to identify the procedure for the patient.

The procedure may be any type of procedure that can be performed within the healthcare facility and may, accordingly, include procedures that take a few minutes to procedures that take days, months, or even longer. For example, the procedure may be a blood draw, medical testing, surgery, in-patient procedure, out-patient procedure, observation stay, recovery stay, consultation, and/or the like. As previously noted, the patient may be undergoing multiple procedures during the same stay at the healthcare facility. In this case, the system identifies all the different procedures that are expected to be performed on the patient.

In addition to those procedures that may be specifically identified, the scheduling system may also be able to identify other procedures for the patient based upon the procedure the patient is presenting for. For example, some procedures may have procedures that are required before a procedure can be performed, some procedures may have sub-procedures, and/or the like. As an example, if a patient is presenting for a surgery, before the surgery is performed the patient may need a particular test performed. As another example, within a testing procedure may be specific tests that need to be performed. In this case, the system can analyze the procedure and identify any other procedures that need to be performed on the patient during the stay. Part of this analysis may include accessing a database that includes listings of procedures and associated procedures, including sub-procedures and dependent procedures.

Part of the analysis may be performed using one or more machine-learning models. A machine-learning model, which may be a neural network, decision tree and/or forest, classifiers, random tree forest or classifier, a combination thereof, a combination of machine-learning models, and/or the like, may be utilized in performing one or more acts of the described system. For example, one or more machine-learning models can be used to identify events of a procedure, identify a granularity to schedule events, generate the schedule, optimize the schedule, and/or the like. It should be understood that while the terminology may refer to a single machine-learning model, multiple machine-learning models can be utilized in performing one or more functions of the scheduling system. The machine-learning model may include a plurality of layers, including input, output, hidden, a combination thereof, and/or the like, layers. The machine-learning model is very complex and utilizes complicated mathematical computations. Due to the complexity of the machine-learning model, it would be impossible to perform the analysis as performed by the model in the human mind.

Additionally, the machine-learning model is trained to or utilized to make predictions on data that has been previously unseen by the model. To make these predictions, the model includes very complicated mathematical computations that would not be performed in the human mind. Rather, the use of a computer and processor, and, possibly a computer and processor that is specific and tuned to the machine-learning model, allows for performing these complex computations, particularly with a speed that allows for performing the complex processing found in and required by the machine-learning model in a time frame that facilitates the use of the machine-learning model for making the predictions. This speed is not possible with a human or even a group of humans. Thus, a human or even a group of humans, even using pen and paper, could not perform the analysis performed by the machine-learning model in a manner that would actually result in making the predictions provided by the machine-learning model on the large amount of data that is received by the scheduling system in a length of time that would make the scheduling system function as intended.

The machine-learning model may be trained using a training dataset having annotated training data. Annotated training data includes data that the model may make a prediction upon where the data is annotated with the correct prediction. The machine-learning model can learn from the training dataset how data should be classified or the predictions that should be made with respect to particular data. As predictions are made upon non-annotated data, feedback may be provided. Feedback may be provided in the form of a user making a correction, a user providing input regarding the prediction, predictions from other models regarding the same data, and/or the like. The feedback can be automatically ingested by the model to further train the model, thereby making the model more accurate over time. It should be noted that the model can monitor the predictions made by the model to identify the feedback so that a user does not have manually provide the feedback to the model. Thus, while the model may initially be trained with a training dataset, the model can continually be trained as it is deployed using predictions and feedback regarding the predictions. In the case of the procedures and identification of associated procedures, the machine-learning model may be trained using procedure data and may, therefore, be able to identify associated procedures. Similar training and corresponding predications may be used for other portions of the described system. The machine-learning model also be trained using unsupervised learning techniques, other supervised learning techniques, reinforcement training, a combination thereof, and/or the like.

At 103, the scheduling system identifies a plurality of events corresponding to the at least one procedure and locations within the healthcare facility that correspond to one or more of the events. Events or steps within the procedure are those tasks that need to be performed in order to facilitate the performance of the procedure. To identify the events, the scheduling system may access a procedure system that identifies different events within a procedure. The procedure system may include other components that are also accessible by the scheduling system. For example, the procedure system may include an associated database that includes information related to different procedures, including events within a procedure. The scheduling system may access the procedure system and/or database of the procedure system, locate the procedure within the system, and then access the information of the procedure, including the events of the procedure. The information also identifies a workflow of the procedure. Thus, not only does the information identify what events need to be performed, but also the order in which the events need to be performed.

The information may also identify other attributes of the procedure and/or events of the procedure, for example, a type of healthcare professional responsible for a particular event, the department responsible for a particular event, a type of location needed for performing the event, healthcare objects needed for performing the event, support personnel or department needed to perform the event, and/or the like. It should be understood that some events may require the use of multiple healthcare professionals, support personnel or departments, healthcare objects, locations, and/or the like. Another technique for identifying the events include analyzing events of other similar procedures that have already been scheduled and performed. In other words, the scheduling system may access historical procedures and identify the events that were identified for those procedures. Another technique for identifying the events includes receiving user input. The system may also use a combination of techniques to identify the events. Other techniques for identifying the events are possible and contemplated and the described techniques are intended to be non-limiting.

The granularity at which the events are identified for a procedure may be based upon a default setting, user preferences, learned over time by the scheduling system using machine-learning techniques, and/or the like. For example, the system may learn, using a machine-learning technique or other learning algorithm, that a length of time for a particular step of a procedure varies greatly among patients. In this case, the system identifies this step as a step that should be specifically identified and scheduled due to the variation in the length of time the step can take. On the other hand, the system may learn that a length of time for a particular set of steps are fairly consistent between patients. In this case, the system may group these set of steps to be scheduled as a single event.

The granularity of steps may also be dependent on attributes associated with a step or event. Attributes may include a location of the step, a healthcare professional performing the step, a support personnel or department performing a step, and/or the like. Depending on the perspective of the schedule, meaning who or what the schedule is being generated for (e.g., the patient schedule, the location schedule, the healthcare professional schedule, the support personnel or department schedule, etc.), the scheduling system may schedule events based upon the attributes. For example, if one step is performed in one location and the next step in a different location, the scheduling system may keep these as two separate steps for scheduling location. On the other hand, if one step is performed in one location and the next step is in the same location, the scheduling system may group these as a single step for scheduling the location. Conversely, if in the above example, when scheduling the healthcare professional, if the first step and second step are performed by the same professional, the scheduling system may group the steps together in the healthcare professional schedule. However, if the healthcare professional wanted the steps broken out, the scheduling system could do that also. Thus, the granularity of the schedule may be based upon a combination of factors, for example, user preferences, step attributes, machine-learning, and/or the like.

In addition to identifying the steps or events, the scheduling system also identifies locations for at least some of the events. Identifying the location may be performed by accessing the information contained within the procedure system, identifying historical locations for similar historical procedures, learned using a machine-learning technique, receiving user input, and/or the like. Whether the location is identified may be dependent on whether the location needs to be scheduled. For example, events occurring in an operating room need to be scheduled with the operating room, but events occurring at a registration desk may not need to be scheduled at the registration desk. Different events may be performed in different locations of the healthcare facility, for example, different rooms, different departments, different facilities, and/or the like. For example, a procedure may include a registration step performed at a registration desk or department, a pre-procedure preparation step performed within a preparation room, a procedure step performed in a procedure room, a recovery step performed within a recovery department, and a discharge step associated with a discharge department. The events or steps can also be broken into sub-steps or sub-events, and each of the sub-steps or sub-events may be associated with a particular location, for example, the recovery step may include a post-procedure recovery step associated with a short-term recovery location, an acute recovery step associated with an acute recovery center, a long-term recovery step associated with a long-term recovery facility, and/or the like.

It should be noted that not all locations have to be located on the same healthcare facility grounds. For example, different steps may be associated with facilities that are located in different geographical locations. However, even with locations in different geographical locations or facilities, the scheduling system can still obtain information and generate a schedule for those locations, patients within those locations, healthcare professionals within those locations, support personnel or departments within those locations, and/or the like.

At 104, the scheduling system may identify at least one healthcare professional (e.g, surgeon, doctor, nurse, triage, specialist, physician's assistant, etc.) associated with at least one of the events. Identifying the at least one healthcare professional can be performed using one of the previously described techniques, for example, user input, machine-learning, accessing information from the procedures system, and/or the like, or a combination thereof. In the case that the scheduling system accesses the information from the procedures system, the information may not have a specific healthcare professional identified. Rather, the information may only identify a type of healthcare professional, qualifications for the healthcare professional, a role of the healthcare professional, and/or other attributes of the healthcare professional. However, in order to accurately schedule a healthcare professional, the scheduling system may need to identify a particular healthcare professional.

To identify a particular healthcare professional from the information, the scheduling system may identify one or more possible healthcare professionals that fulfill the attributes identified within the information. The scheduling system may then correlate these possible healthcare professionals against other information to identify a healthcare professional that can be scheduled at the time. The other information may include a healthcare professional schedule which identifies when healthcare professionals are scheduled to be within a particular facility or location, healthcare professional on-call schedules, healthcare professional availability, healthcare professional location, healthcare professional historical schedules, and/or the like. Thus, the scheduling system can output one or more possible healthcare professionals that could be scheduled for the step within the procedure. This continues until all healthcare professionals that are needed to complete all steps of the procedure have been identified.

Similarly, the scheduling system may identify healthcare objects (e.g., testing equipment, patient bed, intravenous bags, surgical equipment, etc.), support personnel or departments (e.g., transport, registration, janitorial, management, housekeeping, etc.), and/or the like, that are associated with the events of the procedure. The identification of these objects, people, departments, and/or the like, can be performed in a similar manner as the identification of location and/or healthcare professional.

At 105, the scheduling system determines whether a schedule can be automatically generated for the healthcare facility based upon the plurality of events and healthcare professionals. To determine whether a schedule can be automatically generated, the scheduling system may attempt to generate the schedule. Generating the schedule includes generating at least one schedule for the healthcare facility. The perspective of the schedule may vary depending on the user requesting the schedule, the user viewing the schedule, a schedule configuration programmed in the scheduling system, and/or the like. The perspective of the schedule means the part or person of the healthcare facility that the schedule corresponds to. For example, the perspective may be a particular healthcare professional, a location within the healthcare facility, a patient, a support personnel or department, a healthcare object, and/or the like. As an example, the scheduling system can generate a schedule for a healthcare professional. As another example, the scheduling system can generate a schedule for a particular patient. As a final, non-limiting example, the scheduling system can generate a schedule for a support department.

The time frame of the schedule is configurable and may vary based upon different criteria, for example, user configuration, role of a user viewing the schedule, a default configuration, and/or the like. Example time frames include a shift schedule, a daily schedule, a weekly schedule, a rotation schedule, a monthly schedule, a procedure timeframe schedule, and/or the like. The system may also generate schedules with different time frames for different users, different healthcare objects, different locations, different support personnel or departments, and or the like. For example, the scheduling system may generate a daily schedule for locations within the healthcare facility, but generate a procedure timeframe schedule for a patient.

When generating schedules for people, departments, objects, and/or the like, other than the patient, the system may take into account procedure information received from multiple patients. In other words, to generate a shift schedule for a healthcare professional, the scheduling system would need to take into account all patients that the healthcare professional will interact with during the shift. Similarly, for locations, healthcare objects, support personnel or departments, and the like, the scheduling system would take into account all patients that will interact with or cause a need for a location, healthcare object, support personnel or department, and/or the like, when generating the corresponding schedule.

In generating the schedule, the system is attempting to optimize the scheduling for the corresponding healthcare facility object, person (e.g., healthcare professional, patient, support personnel or department, etc.), location, and/or the like. This means that the scheduling system attempts to minimize deadtime (i.e., time that is not being utilize for performing or supporting a procedure) and schedule overlaps (i.e., time that is double-booked). To this end, the scheduling system attempts to identify how long each step or event of the procedure will take with respect to each patient. In other words, the scheduling system attempts to identify how long each step or event will take for a particular patient, thereby allowing for accurate scheduling of resources (e.g., healthcare professionals, healthcare objects, support personnel or departments, locations, etc.) used to facilitate the performance of the procedure for the patient. Accordingly, to optimize the schedule, the scheduling system may utilize one or more techniques to more accurately perform the scheduling.

One technique for optimizing the scheduling is to utilize historical information associated with the patient to generate the schedule. The scheduling system may access historical procedures that are associated with the patient to determine if the patient has had a procedure similar to the procedure for which the patient is currently presenting at the healthcare facility, referred to as the target procedure for ease of understanding. A similarity may be determined using one or more similarity measurement techniques and a procedure may be identified as similar if the similarity reaches or exceeds a predetermined similarity threshold. For example, if the target procedure is cataract surgery, the scheduling system may determine whether the patient has previously had cataract surgery. If a similar procedure is identified, the scheduling system may access the information associated with that historical procedure, for example, historical schedule information, average procedure information, and/or the like, to identify how long each step or event took for the historical procedure. Based upon this historical information, the scheduling system can then appropriately schedule the events for the target procedure.

If a similar procedure cannot be identified, for example, because the patient has never had such a procedure performed, the scheduling system may try to identify historical procedures that the patient has had performed and identify if the historical procedures have any similar steps or events to the target procedure. If the scheduling system identifies a historical procedure having a similar step or event, for example, a similar event above a predetermined threshold, the scheduling system may identify the length of time the step took in the historical procedure and then assign the same length of time to the similar step within the target procedure. The scheduling system may continue to do this analysis for each step within the target procedure. In a similar manner, optimization of a schedule for a particular healthcare resource can be performed using historical information for the healthcare resource or a similar healthcare resource.

In the event of multiple similar historical procedure or multiple similar historical procedure steps, the system may take an average of the historical procedures or historical procedure steps, may identify a most commonly occurring time, identify a most recent occurring time, may choose the longest occurring time, and/or the like, or a combination thereof. The scheduling system may also take into account other factors that may have contributed to a particular time and determine if similar factors are present for the target procedure. In this case, the system may then attribute the time of the historical procedure or historical procedure steps having similar factors to the target procedure or target procedure steps.

In addition to the patient historical procedure information, the scheduling system may take into characteristics of the patient and identify other patients that have characteristics similar to the patient and that have had a similar procedure or procedure step performed. For example, in the event that the patient has no historical procedure information or no historical procedure information similar to the target procedure, the scheduling system can identify other patients who have undergone a similar procedure or procedure steps to identify a length of time of those similar patients. Characteristics that may be taken into account may include those characteristics that can effect procedure times, for example, gender, age, healthcare risk factors, allergies, special accommodations, geographical region, and/or the like.

The scheduling system may also use this type of crowd-sourced information in order to more accurately identify a length of time for a procedure or procedure step, validate procedure or procedure step times of the patient that were identified from historical procedures of the patient, and/or the like. In other words, the crowd-sourced information may be used even if the patient has similar historical procedure or procedure step information.

Another factor that may affect a procedure or procedure step length is the resource that is used for performing the procedure or procedure step. For example, one piece of testing equipment may be slower than another piece of testing equipment that performs the same test. Accordingly, the piece of testing equipment that is used will change the length of a procedure step. As another example, a particular healthcare professional may perform a procedure in a different length of time than another healthcare professional. Accordingly, the healthcare professional performing the procedure will change the length of a procedure step. Thus, the scheduling system may take into account the differences in the resource (e.g., healthcare object, healthcare professional, support personnel or department, location, etc.) when optimizing the schedule. In a similar manner to that of the patient, the scheduling system can access historical procedures to identify a length of time that resulted for a procedure or procedure step when using a particular resource.

Another technique that may be used when optimizing the schedule is the utilization of one or more machine-learning models. In this case, the machine-learning model(s) are trained using historical procedure and procedure step information. The trained machine-learning model can be deployed to make predications regarding a length of time that will be required to perform a procedure or a procedure step. As new predictions are made and the procedures and procedure steps are performed, the model(s) can identify how long the procedure and procedure steps actually took and use this information as feedback to retrain, optimize, or otherwise make the model(s) more accurate with respect to subsequent predictions. Other techniques and combinations of techniques for generating the schedule are contemplated and possible.

It should be noted that while it is discussed that the scheduling system attributes historical procedure time information to the target procedure, the scheduling system may also add a buffer amount of time in order to account for any possible variations between the historical procedure or procedure steps and the target procedure or procedure steps. The length of a buffer time may be a default buffer value, a user configured buffer value, a learned buffer value, a buffer value that is dependent on the procedure or procedure step, and/or the like.

Additionally, it should be understood that while the above discussion mostly focused on generating a schedule for a patient, the same techniques can be utilized when generating a schedule for a healthcare resource. To generate the schedule, the system may iteratively perform one or more of the described steps for all patients, healthcare professionals, support personnel or departments, locations within the healthcare facility, healthcare resources, and/or the like, through a particular time period, for example, a few hours, a shift, a day, a few days, a week, a month, and/or any other time frame which can be a default time frame or selected by a user.

If a schedule cannot be automatically generated, the scheduling system may notify the user at 107. The scheduling system may be unable to generate a schedule when scheduling conflicts occur, a number of procedures or procedure steps for a particular healthcare resource exceeds a given timeframe, a healthcare resource becomes unavailable, and/or the like. The scheduling system may also attempt to schedule as much as it can and then provide the user with an indication of those procedures or procedure steps that cannot be scheduled. When notifying the user, the scheduling system may identify what occurred to cause the scheduling system to be unable to generate the schedule.

On the other hand, if the scheduling system identifies that a schedule can be automatically generated, the scheduling system automatically generates a schedule at 106. If any errors, alerts, and/or the like, occur during the generation of the schedule but the schedule is still able to be generated, the scheduling system may provide those errors, alerts, notifications, and/or the like, with the generated schedule.

As previously mentioned, the scheduling system may generate different schedules for different perspectives, for example, the patient, healthcare resource, management, and/or the like. However, different perspectives may want information in addition to the schedule. Thus, in addition to the schedule, the system may also generate other outputs based upon the perspective. One piece of additional information that may be generated by the scheduling system is statistics related to procedures, procedure steps, healthcare resources, and/or the like. For example, management may want to see an efficiency statistic associated with a healthcare resource, differences in times for procedures or procedures steps for different healthcare resources, a historical usage of a healthcare resource, and/or the like. This information can be easily generated by the scheduling system and may assist in determining whether the healthcare facility needs to make modifications regarding different procedures, procedures steps, healthcare resources, and/or the like.

Another piece of additional information that may be generated is a checklist for a healthcare professional that is based upon the schedule for the healthcare resource. The checklist can identify tasks and objects that are needed to fulfill the schedule and perform the procedure and/or procedure steps. Additionally, the checklist may identify a time that the tasks and/or objects need to be completed or ready by in order to not delay the procedure or procedure step. The checklist may be generated for a particular healthcare resource, meaning that only tasks or steps that utilize the healthcare resource are provided on the checklist. Other additional information is possible and contemplated.

All of the outputs, including the schedule and additional information, may be output to one or more display devices. Additionally, the schedule and additional information may be output within the healthcare scheduling application. Additionally, the schedule and additional information may be transmitted to other devices, for example, devices associated with a healthcare resource. In this case, the schedule and additional information may be encrypted for transmission to the other devices. Additionally, when accessing the application, schedule, and/or additional information, the user may have to provide authentication information. The scheduling system may also be connected to other devices that can generate outputs, for example, printers, facsimile machines, and/or the like. These devices may then provide a hardcopy of the generated schedule, additional information, and/or the like.

The scheduling system may also generate notifications with respect to the schedule and additional information. For example, when schedules or additional information are transmitted to user devices, the system may also provide a notification indicating that the schedule and/or additional information is available for viewing. The scheduling system may also provide notifications when procedure steps are nearing completion, for example, for subsequent procedure step resources, when procedure steps should be started, when healthcare resources need to be ready for performing a procedure or procedure step, and/or the like.

In addition to notifications for healthcare resources, the scheduling system may generate notifications for users associated with a particular patient, for example, an emergency contact, a person identified as being able to receive information related to the patient, a person responsible for the patient, and/or the like. The notifications provided to the users associated with the patient may include notifications that provide information regarding the progress of the procedure for the patient. Non-limiting examples of information that may be transmitted include notifications indicating a transition of a patient from one procedure step to another, healthcare professional notes regarding the procedure or a procedure step, an estimated time for completion of a procedure or procedure step, an alert indicating the patient is ready for discharge or visitors, a current location of the patient, an expected length of time at the current location, and/or the like. At least a portion of this information can be identified from the generated schedule(s).

In order to transmit information to the user(s) associated with the patient, the user(s) may have to provide personal information, for example, device information for transmitting the notification, authorization for receipt of the information, and/or the like. Thus, the healthcare facility can ensure the privacy of the patient. Additionally, the patient may identify how much information can be transmitted to the user(s) associated with the patient.

While various other circuits, circuitry or components may be utilized in information handling devices, with a computer, server, client device or the like, an example device that may be used in implementing one or more embodiments includes a computing device in the form of a computer 10' as illustrated in FIG. 2. This example device may be a server used in one of the systems in a hospital network, or one of the remote computers connected to the hospital network. Components of computer 10' may include, but are not limited to, a processing unit 20', a system memory 30', and a system bus 22' that couples various system components including the system memory 30' to the processing unit 20'. Computer 10' may include or have access to a variety of computer readable media, including databases. The system memory 30' may include non-signal computer readable storage media, for example in the form of volatile and/or nonvolatile memory such as read only memory (ROM) and/or random access memory (RAM). By way of example, and not limitation, system memory 30' may also include an operating system, application programs, other program modules, and program data.

A user can interface with (for example, enter commands and information) the computer 10' through input devices 50'. A monitor or other type of device can also be connected to the system bus 22' via an interface, such as an output interface 360. The computer may include a database 40'. In addition to a monitor, computers may also include other peripheral output devices. The computer 10' may operate in a networked or distributed environment using logical connections to one or more other remote device(s) 80' such as other computers. The logical connections may include network interface(s) 70' to a network, such as a local area network (LAN), a wide area network (WAN), and/or a global computer network, but may also include other networks/buses.

Information handling device circuitry, as for example outlined in FIG. 2, may be used in client devices such as a personal desktop computer, a laptop computer, or smaller devices such as a tablet or a smart phone. In the latter cases, i.e., for a tablet computer and a smart phone, the circuitry outlined in FIG. 2 may be adapted to a system on chip type circuitry. The device, irrespective of the circuitry provided, may provide and receive data to/from another device, e.g., a server or system that coordinates with various other systems. As will be appreciated by one having ordinary skill in the art, other circuitry or additional circuitry from that outlined in the example of FIG. 2 may be employed in various electronic devices that are used in whole or in part to implement the systems, methods and products of the various embodiments described herein.

As will be appreciated by one skilled in the art, various aspects may be embodied as a system, method or device program product. Accordingly, aspects may take the form of an entirely hardware embodiment or an embodiment including software that may all generally be referred to herein as a "circuit," "module" or "system." Furthermore, aspects may take the form of a device program product embodied in one or more device readable medium(s) having device readable program code embodied therewith.

It should be noted that the various functions described herein may be implemented using instructions stored on a device readable storage medium, such as a non-signal storage device, and that are executed by a processor. A storage device may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random-access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a storage device is not a signal and "non-transitory" includes all media except signal media.

Program code embodied on a storage medium may be transmitted using any appropriate medium, including but not limited to wireless, wireline, optical fiber cable, RF, et cetera, or any suitable combination of the foregoing.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of connection or network, including a local area network (LAN) or a wide area network (WAN), or the connection may be made through other devices (for example, through the Internet using an Internet Service Provider), through wireless connections, e.g., near-field communication, or through a hard wire connection, such as over a USB connection.

Example embodiments are described herein with reference to the figures, which illustrate example methods, devices and program products according to various example embodiments. It will be understood that the actions and functionality may be implemented at least in part by program instructions. These program instructions may be provided to a processor of a device, a special purpose information handling device, or other programmable data processing device to produce a machine, such that the instructions, which execute via a processor of the device implement the functions/acts specified.

It is worth noting that while specific blocks are used in the figures, and a particular ordering of blocks has been illustrated, these are non-limiting examples. In certain contexts, two or more blocks may be combined, a block may be split into two or more blocks, or certain blocks may be re-ordered or re-organized as appropriate, as the explicit illustrated examples are used only for descriptive purposes and are not to be construed as limiting.

As used herein, the singular "a" and "an" may be construed as including the plural "one or more" unless clearly indicated otherwise.

## Claims

1. A method, the method comprising:
obtaining, at a scheduling system of a healthcare facility, patient information for at least one patient, wherein the patient information identifies an identifier of the at least one patient;
identifying, using the scheduling system and based upon the patient information, at least one procedure to be performed for the patient at the healthcare facility;
identifying, using the scheduling system, a plurality of events corresponding to the at least one procedure and locations within the healthcare facility for at least a subset of the plurality of events;
identifying, using the scheduling system, at least one healthcare professional associated with at least one of the plurality of events; and
automatically generating, using the scheduling system, a schedule for the healthcare facility based upon the plurality of events and the at least one healthcare professional.

2. The method of claim 1, wherein the patient information identifies characteristics of the at least one patient.

3. The method of claim 2, wherein the automatically generating comprises identifying historical procedure schedules for patients having a set of characteristics having a similarity to the characteristics of the at least one patient and generating the schedule based upon the historical procedure schedules.

4. The method of claim 1, wherein the automatically generating comprises identifying historical procedures for the at least one patient and generating the schedule based upon the historical procedures for the at least one patient.

5. The method of claim 1, wherein the generating a schedule for the healthcare facility comprises generating a schedule for the patient.

6. The method of claim 1, wherein the generating a schedule for the healthcare facility comprises generating a schedule for at least one of: a healthcare professional and a location within the healthcare facility based upon patient information for a plurality of patients.

7. The method of claim 6, wherein the generating a schedule comprises optimizing the schedule for the at least one of a healthcare professional and a location based upon historical procedure information and schedules.

8. The method of claim 1, further comprising generating and transmitting a checklist to at least one healthcare professional based upon the schedule, wherein the checklist identifies at least one of: tasks and objects needed to fulfill the schedule and a time of need of the at least one of tasks and objects.

9. The method of claim 1, further comprising generating and transmitting automatic notifications to a user device corresponding to a person associated with the at least one patient, wherein the automatic notifications provide information identifying a current location of the at least one patient and an expected length of time at the current location as identified from the schedule.

10. The method of claim 1, further comprising providing an application for accessing the scheduling system and providing within a graphical user interface of the application information associated with the schedule.

11. A system, the system comprising:
a processor;
a memory device that stores instructions that, when executed by the processor, causes the system to carry out a method according to any one of the preceding claims.

12. A product, the product comprising:
a computer-readable storage device that stores executable code that, when executed by a processor, causes the product to:
obtain, at a scheduling system of a healthcare facility, patient information for at least one patient, wherein the patient information identifies an identifier of the at least one patient;
identify, using the scheduling system and based upon the patient information, at least one procedure to be performed for the patient at the healthcare facility;
identify, using the scheduling system, a plurality of events corresponding to the at least one procedure and locations within the healthcare facility for at least a subset of the plurality of events;
identify, using the scheduling system, at least one healthcare professional associated with at least one of the plurality of events; and
automatically generate, using the scheduling system, a schedule for the healthcare facility based upon the plurality of events and the at least one healthcare professional.
